# EUROPEAN PATENT APPLICATION

(11) **EP 4 733 402 A1**
(43) Date of publication of application: **29.04.2026**
(21) Application number: 24858657.0
(22) Date of filing: 29.08.2024
(51) Int. Cl.: C12N 15/82, A01H 6/46, C07K 14/415

(54) **METHOD FOR IMPROVING INSECT RESISTANCE AND DISEASE RESISTANCE OF PLANT**

(30) Priority: 29.08.2023 CN 202311094713
(71) Applicant: Shandong Shunfeng Biotechnology Co., Ltd., Jinan, Shandong 250000 (CN)
(72) Inventor: XIE, Guangning, Jinan, Shandong 250000 (CN); LIU, Hui, Jinan, Shandong 250000 (CN)
(74) Representative: Bayramoglu et al.
(86) International application number: PCT/CN2024/115340
(87) International publication number: WO 2025/045125

(57) **Abstract**

The present disclosure discloses a method for improving a trait of a plant, including a step of mutating a BX13 gene and a BX14 gene. The mutation of the BX13 gene and the BX14 gene results in enhanced pest resistance, enhanced disease resistance, an increased ear size, an increased kernel size, an increased hundred-kernel weight, and an elevated yield of the plant, demonstrating significant application value in plant breeding.

## Description

The present application claims priority to the Chinese Patent Application CN202311094713.4 filed on August 29, 2023, which is incorporated herein by reference in its entirety.

### TECHNICAL FIELD

The present disclosure belongs to the fields of biotechnology and crop genetic breeding, and relates to a method for improving pest resistance, disease resistance, and a yield of corn. In particular, the present disclosure relates to a method for improving pest resistance and disease resistance of corn, increasing an ear size and a kernel size of the corn, increasing a hundred-kernel weight of the corn, or boosting a yield of the corn through a mutation in BX13 and BX14 genes.

### BACKGROUND TECHNOLOGY

Corn, as an important source of food, feed, and industrial raw materials, holds a significant position in global grain production. Due to the occurrence of diseases and pests in corn, the corn production has been greatly affected. Currently, major corn pests include armyworms, corn borers, fall armyworms, and corn earworms, and common corn diseases include ear rot, stalk rot, northern corn leaf blight, and southern rust.

To enhance the pest and disease resistance of corn, the relevant genes in corn have been investigated to broaden the spectra of resistance to pests and pathogens in corn, thereby producing corn lines with superior traits.

### CONTENT OF THE INVENTION

An objective of the present disclosure is to provide a method for improving a trait of a plant.

A first aspect of the present disclosure provides a method for improving a trait of a plant, including a step of mutating a BX13 gene and/or a BX14 gene.

In another preferred embodiment, an amino acid sequence encoded by the BX13 gene is selected from the group consisting of:
(i) a polypeptide with an amino acid sequence shown in SEQ ID NO.: 1;
(ii) a polypeptide that is produced through substitution, deletion, or addition of one or more (such as 1 to 10) amino acid residues in the amino acid sequence shown in SEQ ID NO.: 1 and has the same or similar function as or to the polypeptide defined in the (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 1 and demonstrates the same or similar function as or to the amino acid sequence shown in SEQ ID NO.: 1.

In another preferred embodiment, an amino acid sequence encoded by the BX14 gene is selected from the group consisting of:
(i) a polypeptide with an amino acid sequence shown in SEQ ID NO.: 2;
(ii) a polypeptide that is produced through substitution, deletion, or addition of one or more (such as 1 to 10) amino acid residues in the amino acid sequence shown in SEQ ID NO.: 2 and has the same or similar function as or to the polypeptide defined in the (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 2 and demonstrates the same or similar function as or to the amino acid sequence shown in SEQ ID NO.: 2.

In another preferred embodiment, a nucleotide sequence of the BX13 gene is selected from the group consisting of:
(a) a polynucleotide encoding the polypeptide shown in SEQ ID NO.: 1;
(b) a polynucleotide with a sequence shown in SEQ ID NO.: 3;
(c) a polynucleotide that has a nucleotide sequence possessing a homology of 95% or more (preferably 98% or more and more preferably 99% or more) with the sequence shown in SEQ ID NO.: 3;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 3; and
(e) a polynucleotide complementary to a polynucleotide defined in any of the (a) to the (d).

In another preferred embodiment, a nucleotide sequence of the BX14 gene is selected from the group consisting of:
(a) a polynucleotide encoding the polypeptide shown in SEQ ID NO.: 2;
(b) a polynucleotide with a sequence shown in SEQ ID NO.: 4;
(c) a polynucleotide that has a nucleotide sequence possessing a homology of 95% or more (preferably 98% or more and more preferably 99% or more) with the sequence shown in SEQ ID NO.: 4;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 4; and
(e) a polynucleotide complementary to a polynucleotide defined in any of the (a) to the (d).

In another preferred embodiment, the amino acid sequence encoded by the BX13 gene has a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with SEQ ID No. 1. In another preferred embodiment, the amino acid sequence encoded by the BX13 gene is shown in SEQ ID No. 1. In another preferred embodiment, an accession number of the BX13 gene is Zm00001d007718.

In another preferred embodiment, the amino acid sequence encoded by the BX14 gene has a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with SEQ ID No. 2. In another preferred embodiment, the amino acid sequence encoded by the BX14 gene is shown in SEQ ID No. 2. In another preferred embodiment, an accession number of the BX14 gene is Zm00001d004921.

In another preferred embodiment, improving the trait of the plant is selected from one or more of the following (i) to (vi):
(i) enhancing pest resistance of the plant;
(ii) enhancing disease resistance of the plant;
(iii) increasing an ear size;
(iv) increasing a kernel size;
(v) increasing a hundred-kernel weight; and
(vi) boosting a yield of the plant.

In another preferred embodiment, enhancing the pest resistance of the plant includes an increase in resistance of the plant to a chewing pest, a sucking pest, or a boring pest.

In another preferred embodiment, enhancing the pest resistance of the plant includes an increase in resistance of the plant to the chewing pest.

In another preferred embodiment, the chewing pest includes an armyworm, a fall armyworm, a corn borer, a wheat sawfly, a locust, a yellow rice borer, a pink borer, a cutworm, a corn earworm, or a cabbage worm.

In another preferred embodiment, the chewing pest includes the armyworm, the fall armyworm, or the corn borer.

In another preferred embodiment, the chewing pest includes the armyworm.

In another preferred embodiment, enhancing the pest resistance of the plant includes an increase in resistance of the plant to the armyworm.

In another preferred embodiment, enhancing the disease resistance of the plant includes an increase in resistance of the plant to stalk rot.

In another preferred embodiment, enhancing the disease resistance of the plant includes an increase in resistance of the plant to a pathogen associated with the stalk rot.

In another preferred embodiment, enhancing the disease resistance of the plant includes an increase in resistance of the plant to *Fusarium graminearum, Fusarium moniliforme, Penicillium, Aspergillus, Cladosporium,* or *Trichothecium.*

In another preferred embodiment, enhancing the disease resistance of the plant includes an increase in resistance of the plant to the *Fusarium graminearum.*

In another preferred embodiment, the mutating is achieved by a technology selected from the group consisting of gene mutation, gene knockout, gene disruption, an RNA interference technology, a gene editing technology, introduction of an inhibitor for a gene or a protein, or a combination thereof.

In another preferred embodiment, the gene mutation is achieved through one or more of the following approaches: natural variation, physical mutagenesis (such as ultraviolet-induced mutagenesis, X-ray-induced mutagenesis, or γ-ray-induced mutagenesis), chemical mutagenesis (such as nitrous acid, hydroxylamine, ethyl methanesulfonate (EMS), and nitrosoguanidine), biological mutagenesis (such as virus or bacterium-mediated mutagenesis), gene editing, or biosynthesis.

In another preferred embodiment, the gene editing technology is selected from the group consisting of a clustered regularly interspaced short palindromic repeat (CRISPR) technology, a transcription activator-like effector nuclease (TALEN) technology, a zinc finger nuclease (ZFN) technology, or a combination thereof. In another preferred embodiment, the method includes administering an inhibitor for the BX13 gene and/or the BX14 gene.

In another preferred embodiment, the inhibitor is selected from the group consisting of an antisense nucleic acid, an antibody, a small-molecule compound, a Crispr reagent, a small-molecule ligand, or a combination thereof.

In another preferred embodiment, the method includes the following steps:
(i) providing a plant or a plant cell; and
(ii) introducing a guide RNA (gRNA) targeting the BX13 gene and/or the BX14 gene and a corresponding Cas protein into the plant or the plant cell. In a preferred embodiment, an expression vector carrying the gRNA and the Cas protein is introduced into the plant or the plant cell.

In another preferred embodiment, the Cas protein is Cas9.

In another preferred embodiment, the method includes the following steps:
(i) providing a plant or a plant cell; and
(ii) introducing an inhibitor for the BX13 gene and/or the BX14 gene into the plant or the plant cell to produce a modified plant or plant cell.

In another preferred embodiment, the mutating includes an insertion mutation, a deletion mutation, a frameshift mutation, and a substitution mutation.

In a preferred implementation, the method includes reducing or inhibiting an expression level and/or activity of the BX13 gene and/or the BX14 gene.

In another preferred embodiment, the reducing or inhibiting means that an expression level E1 of the BX13 gene and/or the BX14 gene in the plant is 0% to 80%, preferably 0% to 60%, more preferably 0% to 40%, and even more preferably 0% to 30% of an expression level E0 of the BX13 gene and/or the BX14 gene in a wild-type (WT) plant.

In another preferred embodiment, the reducing or inhibiting an expression level and/or activity of the BX13 gene and/or the BX14 gene is achieved by a technology selected from the group consisting of gene mutation, gene knockout, gene disruption, an RNA interference technology, a gene editing technology, introduction of an inhibitor for a gene or a protein, or a combination thereof.

In another preferred embodiment, the mutating results in a complete or partial loss of a function of the BX13 gene, and preferably, the mutating results in a complete loss of the function of the BX13 gene.

In another preferred embodiment, the mutating results in a complete or partial loss of a function of the BX14 gene, and preferably, the mutating results in a complete loss of the function of the BX14 gene.

In another preferred embodiment, the mutating includes deletion of some bases in a nucleotide sequence of the BX13 gene that is shown in SEQ ID NO. 3. In another preferred embodiment, the mutating includes deletion of 242 bases after base 144 in the nucleotide sequence of the BX13 gene that is shown in SEQ ID NO. 3.

In another preferred embodiment, the mutating includes deletion of some bases in a nucleotide sequence of the BX14 gene that is shown in SEQ ID NO. 4. In another preferred embodiment, the mutating includes deletion of 2 bases after base 572 in the nucleotide sequence of the BX14 gene that is shown in SEQ ID NO. 4. In another preferred embodiment, the mutating includes deletion of 197 bases after base 376 in the nucleotide sequence of the BX14 gene that is shown in SEQ ID NO. 4.

In another preferred embodiment, the mutating includes insertion of one or more bases in the nucleotide sequence of the BX13 gene that is shown in SEQ ID NO. 3. In another preferred embodiment, the mutating includes insertion of one base after base 145 in the nucleotide sequence of the BX13 gene that is shown in SEQ ID NO. 3.

In another preferred embodiment, the mutating includes insertion of one or more bases in the nucleotide sequence of the BX14 gene that is shown in SEQ ID NO. 4.

In another preferred embodiment, a nucleotide sequence of a mutated BX13 gene is shown in SEQ ID NO. 5 or SEQ ID NO. 7.

In another preferred embodiment, a nucleotide sequence of a mutated BX14 gene is shown in SEQ ID NO. 6 or SEQ ID NO. 8.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae, and Arecaceae* plants.

In another preferred embodiment, the plant is one or more plants selected from the group consisting of soybean, *Arabidopsis thaliana,* rice, tobacco, tomato, potato, corn, cotton, alfalfa, sorghum, barley, wheat, millet, sweet potato, quinoa, lettuce, rapeseed, *Brassica rapa,* spinach, beet, peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is corn.

In another preferred embodiment, the corn is a female parent W67 of Wan Nuo 2000.

A second aspect of the present disclosure provides a composition for improving a trait of a plant, including:
(a) an inhibitor for a BX13 gene and/or a BX14 gene; and optionally,
(b) an agriculturally acceptable carrier.

In another preferred embodiment, an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

In a preferred embodiment, the inhibitor can reduce or inhibit an expression level and/or activity of the BX13 gene and/or the BX14 gene.

In another preferred embodiment, the composition includes an agricultural composition.

In another preferred embodiment, the inhibitor includes an agricultural inhibitor.

In another preferred embodiment, a dosage form of the composition is selected from the group consisting of a solution, an emulsion, a suspension, a powder, a foam, a paste, a granule, an aerosol, or a combination thereof.

In another preferred embodiment, the inhibitor is selected from the group consisting of a gene editing reagent, an antisense nucleic acid, an antibody, a small-molecule compound, a Crispr reagent, a small-molecule ligand, or a combination thereof.

In an embodiment, the gene editing reagent includes a Cas enzyme and gRNA capable of targeting a target gene.

In another preferred embodiment, the antisense nucleic acid is selected from the group consisting of antisense RNA, antisense DNA, interfering RNA, a ribozyme, or a combination thereof.

In another preferred embodiment, the interfering RNA is selected from the group consisting of small interfering RNA (siRNA), short hairpin RNA (shRNA), RNA interference (RNAi), microRNA (miRNA), double-stranded RNA (dsRNA), hairpin RNA (hpRNA), intron-containing hairpin RNA (ihpRNA), or a combination thereof.

In another preferred embodiment, the composition further includes other substances capable of improving the trait of the plant.

In another preferred embodiment, the composition further includes other substances capable of enhancing pest resistance and/or disease resistance of the plant.

A third aspect of the present disclosure provides a use of the composition described in the second aspect of the present disclosure in improvement of a trait of a plant.

In another preferred embodiment, the use of the composition includes a use in preparation of a reagent or kit for producing a plant with an improved trait.

A fourth aspect of the present disclosure provides a method for producing a plant cell or a plant seed or a plant tissue or a plant part or a plant with an improved trait, including the following step:
reducing or inhibiting an expression level and/or activity of a BX13 gene and/or a BX14 gene in a plant cell or a plant seed or a plant tissue or a plant part or a plant to produce the plant cell or the plant seed or the plant tissue or the plant part or the plant with the improved trait.

In another preferred embodiment, an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

A fifth aspect of the present disclosure provides a method for producing a plant with an improved trait, including the following step:
regenerating a plant from a plant cell or a plant seed or a plant tissue or a plant part with an improved trait produced by the method described in the fourth aspect of the present disclosure to produce the plant with the improved trait.

In another preferred embodiment, the method further includes a step of harvesting a plant seed from the plant with the improved trait.

A sixth aspect of the present disclosure provides a plant cell or a plant seed or a plant tissue or a plant part or a plant with an improved trait that is produced by the method described in the fourth or fifth aspect of the present disclosure.

A seventh aspect of the present disclosure provides a method for improving a trait of a plant, including the following steps:
(a) providing a plant cell, a plant tissue, or a plant part; and introducing an inhibitor for a BX13 gene and/or a BX14 gene into the plant cell, the plant tissue, or the plant part, or reducing or inhibiting an expression level and/or activity of the BX13 gene and/or the BX14 gene in the plant cell, the plant tissue, or the plant part; and
(b) regenerating a plant from a plant cell, a plant tissue, or a plant part obtained in the step (a).

In another preferred embodiment, an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

In another preferred embodiment, in the step (a), the plant cell, the plant tissue, or the plant part is modified by a gene editing technology to reduce an expression level or activity of the BX13 gene and/or the BX14 gene in the plant cell, the plant tissue, or the plant part.

In another preferred embodiment, the gene editing technology is selected from the group consisting of a CRISPR gene editing system, error-prone polymerase chain reaction (PCR), gene recombination, TALEN, and ZFN.

In another preferred embodiment, the improving a trait includes an increase in pest resistance and/or disease resistance.

An eighth aspect of the present disclosure provides a genetically engineered plant produced by the method described in the seventh aspect of the present disclosure.

A ninth aspect of the present disclosure provides a method for screening or identifying pest resistance and/or disease resistance of a plant, including a step of detecting an expression level of a BX13 gene and/or a BX14 gene.

In another preferred embodiment, an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

In another preferred embodiment, a part of the plant to be detected includes a callus, a fruit, a seed, a flower, a stalk, a leaf, an ear, and a root of the plant.

In another preferred embodiment, the plant is corn.

A tenth aspect of the present disclosure provides a method for producing a plant seed with an improved trait, including a step of using a plant produced by the method described above to produce the plant seed with the improved trait.

An eleventh aspect of the present disclosure also provides a plant seed with an improved trait, where the plant seed with the improved trait is produced by the method for producing a plant seed with an improved trait described above.

A twelfth aspect of the present disclosure also provides a method for producing a hybrid plant, including a step of crossing a plant seed with an improved trait or a plant with an improved trait produced by the method described above with another plant to produce the hybrid plant.

A thirteenth aspect of the present disclosure also provides a method for inhibiting growth of a pest or killing the pest, including the following steps:
(a) preparing a plant seed, a plant tissue, a plant part, or a plant with an improved trait by the method described above; and
(b) feeding the pest with the plant seed, the plant tissue, the plant part, or the plant prepared in the step (a).

It should be understood that, within the scope of the present disclosure, the technical features mentioned above and the specific technical features described in detail below (such as in embodiments) in the present disclosure can be combined with each other to constitute new or preferred technical solutions, which are not repeated one by one here for conciseness.

### Specific implementations

Unless defined otherwise, the technical and scientific terms used herein have the same meanings as commonly understood by those of ordinary skill in the art.

As used herein, the terms "polynucleotide", "nucleotide sequence", "nucleic acid sequence", "nucleic acid molecule", and "nucleic acid" can be used interchangeably, and include DNA, RNA, or a hybrid thereof, which may be double-stranded or single-stranded.

The term "homology" or "identity" refers to sequence matching between two polypeptides or between two nucleic acids. When specified positions in two sequences to be compared are occupied by a same base or amino acid monomer subunit (for example, a specified position in each of two DNA molecules is occupied by adenine, or a specified position in each of two polypeptides is occupied by lysine), the molecules are identical at the position. Generally, the comparison is conducted by aligning two sequences to produce the maximum identity. An alignment method is a conventional technique well known to those skilled in the art, such as the basic local alignment search tool (BLAST) algorithm.

The term "genetic engineering" refers to a technology in which nucleotides controlling biological genetic information are modified and utilized through artificial intervention to produce novel genetic traits, varieties, or products. The genetic engineering includes all genetic modification technologies disclosed in the art, such as gene mutagenesis, transgenic technique, or gene editing. A method for the gene mutagenesis includes, but is not limited to, physical mutagenesis (such as ultraviolet-induced mutagenesis), chemical mutagenesis (such as acridine dyes), and biological mutagenesis (such as virus or phage-mediated mutagenesis).

A specific amino acid position (number) in a target protein of the present disclosure is determined by aligning an amino acid sequence of the target protein with any one of SEQ ID Nos. 1-2 using a standard sequence alignment tool. For example, the Smith-Waterman algorithm or the CLUSTALW2 algorithm is used to align two sequences, and the sequences are considered aligned when an alignment score is the highest. The alignment score can be calculated according to the method described in Wilbur, W. J. and Lipman, D. J. (1983) Rapid similarity searches ofnucleic acid and protein data banks. Proc. Natl. Acad. Sci. USA, 80: 726-730. In the ClustalW2(1.82) algorithm, default parameters are preferably adopted: protein gap opening penalty = 10.0; protein gap extension penalty = 0.2; protein matrix = Gonnet; protein/DNA end gap = -1; and protein/DNAGAPDIST = 4. The AlignX program (a part of the vectorNTI group) is preferably used to align an amino acid sequence of a protein of the present disclosure with any one of SEQ ID Nos. 1-2 under default parameters suitable for multiple alignments (gap opening penalty: 10; and gap extension penalty: 0.05) to determine a position of a specific amino acid in the protein.

The term "encoding" refers to an inherent characteristic of a specific nucleotide sequence in a polynucleotide, such as a gene, cDNA, or mRNA, which serves as a template in biological processes for the synthesis of other polymers and macromolecules with defined nucleotide sequences (such as rRNA, tRNA, and mRNA) or defined amino acid sequences and the corresponding biological characteristics. Therefore, if the transcription and translation of mRNA corresponding to a gene produces a protein in a cell or another biological system, the gene encodes the protein.

The term "amino acid" refers to a carboxylic acid with amino. Various proteins in organisms each are composed of 20 essential amino acids.

The terms "protein", "polypeptide", and "peptide" can be used interchangeably in the present disclosure, and refer to a polymer of amino acid residues, including a polymer in which one or more amino acid residues are a chemical analog of a natural amino acid residue. The protein and polypeptide of the present disclosure can be produced through recombination or chemical synthesis.

In the present disclosure, an amino acid residue can be represented by a single letter or three letters, such as: alanine (Ala, A), valine (Val, V), glycine (Gly, G), leucine (Leu, L), glutamine (Gln, Q), phenylalanine (Phe, F), tryptophan (Trp, W), tyrosine (Tyr, Y), aspartic acid (Asp, D), asparagine (Asn, N), glutamic acid (Glu, E), lysine (Lys, K), methionine (Met, M), serine (Ser, S), threonine (Thr, T), cysteine (Cys, C), proline (Pro, P), isoleucine (Ile, I), histidine (His, H), and arginine (Arg, R).

As used herein, the term "regulatory element", also known as "regulation element", is intended to include a promoter, a terminator sequence, a leader sequence, a polyadenylation sequence, a signal peptide coding region, a marker gene, an enhancer, an internal ribosome entry site (IRES), and other expression control elements (for example, a transcriptional termination signal, such as a polyadenylation signal and a poly-U sequence), and the detailed description can be seen in Goeddel, "GENE EXPRESSION TECHNOLOGY: METHODS IN ENZYMOLOGY" 185, Academic Press, San Diego, California (1990). In some cases, a regulatory element includes sequences that guide the constitutive expression of a nucleotide sequence in many types of host cells and sequences that guide the expression of the nucleotide sequence only in some host cells (such as a tissue-specific regulatory sequence). A tissue-specific promoter can mainly guide the expression in a desired tissue of interest, such as muscles, neurons, bones, skin, blood, specific organs (such as liver and pancreas), or specific cell types (such as lymphocytes). In some cases, a regulatory element can also guide the expression in a time-dependent manner (such as in a cell cycle-dependent or developmental stage-dependent manner), which may be or may not be tissue or cell type-specific. In some cases, the term "regulatory element" covers enhancer elements, such as WPRE; a cytomegalovirus (CMV) enhancer; R-U5' fragment in LTR of HTLV-I ((Mol. Cell. Biol., Vol 8 (1): 466-472, 1988); SV40 enhancer; and an intron sequence between exons 2 and 3 of rabbit β-globin (Proc. Natl. Acad. Sci. USA., Vol. 78 (3): 1527-31, 1981).

The term "promoter" has the meaning well known to those skilled in the art, and refers to a non-coding nucleotide sequence that is located upstream of a gene and can promote the expression of a downstream gene. A constitutive promoter is a nucleotide sequence that will result in the generation of a gene product in a cell under most or all physiological conditions of the cell after the promoter is operably linked to a polynucleotide encoding or defining the gene product. An inducible promoter is a nucleotide sequence that will cause the generation of a gene product in a cell basically only when there is an inducer corresponding to the promoter in the cell after the promoter is operably linked to a polynucleotide encoding or defining the gene product. A tissue-specific promoter is a nucleotide sequence that will cause the generation of a gene product in a cell basically only when the cell is a cell of the tissue type corresponding to the promoter after the promoter is operably linked to a polynucleotide encoding or defining a gene product.

The term "nuclear localization sequence" (NLS) is an amino acid sequence that tags a protein for import into a nucleus through nuclear transport, that is, a protein with NLS is transported into a nucleus. Typically, NLS includes positively-charged Lys or Arg residues that are exposed on a surface of a protein. Exemplary NLS includes, but is not limited to, NLSs derived from SV40 large T antigen, EGL-13, c-Myc, and TUS protein.

The term "operably linked" means that a nucleotide sequence of interest is linked to one or more regulatory elements in a manner allowing the expression of the nucleotide sequence (for example, in an *in vitro* transcription/translation system or in a host cell when a vector is introduced into the host cell).

The term "vector" refers to a construct that includes an element allowing the vector to be integrated into a genome of a host cell or to self-replicate within a cell independently of a genome of the cell. The vector may include any element that guarantees the self-replication. The vector usually carries a gene that is not a part of the central metabolism of a cell and is usually in a form of double-stranded DNA. The selection of a vector generally depends on the compatibility of the vector with a host cell into which the vector is to be introduced. When a vector needs to be used, the selection of the vector depends on a method for transforming a host cell well known to those skilled in the art. For example, a plasmid vector can be used.

Suitable vectors in the present disclosure include commercially available plasmids, including, but not limited to: pBR322 (ATCC37017), pKK223-3 (Pharmacia Fine Chemicals, Uppsala, Sweden), GEM1 (Promega Biotec, Madison, WI, USA), pQE70, pQE60, pQE-9 (Qiagen), pD10, psiX174pBluescript II KS, pNH8A, pNH16a, pNH18A, pNH46A (Stratagene), ptrc99a, pKK223-3, pKK233-3, pDR540, pRIT5 (Pharmacia), pKK232-8, pCM7, pSV2CAT, pOG44, pXT1, pSG (Stratagene), pSVK3, pBPV, pMSG, and pSVL (Pharmacia).

The nucleic acid sequence, nucleic acid construct, or expression vector of the present disclosure can be introduced into a host cell through a variety of techniques, including transformation, transfection, transduction, viral infection, gene gun or Ti-plasmid-mediated gene delivery, calcium phosphate transfection, diethylaminoethyl (DEAE)-dextran-mediated transfection, lipofection, electroporation, etc.

The term "plant tissue" or "plant part" includes a plant cell, a protoplast, a plant tissue culture, a plant callus, a plant clump, a plant embryo, a pollen, an ovule, a seed, a leaf, a stalk, a flower, a branch, a seedling, a fruit, a kernel, an ear, a root, a root tip, an anther, etc.

The term "plant cell" should be understood as any cell derived from or found in a plant, and can produce, for example, an undifferentiated tissue such as a callus, a differentiated tissue such as an embryo, a constituent part of a plant, a plant, or a seed.

The term "plant" should be understood as any differentiated multicellular organism capable of conducting photosynthesis, including: crop plants at any mature or developmental stage, especially monocotyledonous or dicotyledonous plants; vegetable crops including artichoke, turnip cabbage, arugula, leek, asparagus, lettuce (such as head lettuce, leaf lettuce, and romaine lettuce), bok choy, malanga, melons (such as cantaloupe, watermelon, crenshaw melon, honeydew melon, and Roman cantaloupe), rapeseed crops (such as Brussels sprout, cabbage, cauliflower, broccoli, *Brassica oleracea* var. acephala, *Brassica oleracea* var. viridis, Chinese cabbage, and bok choy), cardoon, carrot, napa, okra, onion, celery, parsley, chickpea, parsnip, chicory, pepper, potato, gourd (such as zucchini, cucumber, courgette, squash, and pumpkin), radish, dry bulb onion, rutabaga, purple eggplant (also known as eggplant), salsify, escarole, shallot, endive, garlic, spinach, green onion, squash, greens, beets (sugar beets and fodder beets), sweet potato, Swiss chard, wasabi, tomato, turnip, and spices; fruits and/or vine crops such as apple, apricot, cherry, nectarine, peach, pear, plum, prune, cherry, quince, almond, chestnut, hazelnut, pecan, pistachio, walnut, citrus, blueberry, boysenberry, cranberry, currant, loganberry, raspberry, strawberry, blackberry, grape, avocado, banana, kiwi, persimmon, pomegranate, pineapple, tropical fruit, pome, melon, mango, papaya, and lychee; field crops, such as clover, alfalfa, evening primrose, meadowfoam, corn/maize (feed corn, sweet corn, and popcorn), lupulus, jojoba, peanut, rice, safflower, small grain crops (barley, oat, rye, wheat, etc.), sorghum, tobacco, kapok, legumes (beans, lentils, peas, and soybeans), oil-bearing plants (rapeseed, leaf mustard, poppy, olive, sunflower, coconut, castor oil plant, cocoa bean, and peanut), *Arabidopsis*, fiber plants (cotton, flax, and jute), *Lauraceae* (cinnamon or camphor), or a plant such as coffee, sugar cane, tea, and natural rubber plants; and/or bedding plants such as a flowering plant, cactus, a succulent plant, and/or an ornamental plant, and trees such as forests (broad-leaved and evergreen trees, such as conifers), fruit trees, ornamental trees, nut-bearing trees, shrubs, and other seedlings.

The gene editing technology includes CRISPR technology, TALEN technology, and ZFN technology. The CRISPR technology refers to clustered regularly interspaced short palindromic repeats, which come from the immune system of microorganisms. A gene editing tool of the CRISPR technology includes gRNA and Cas protein (such as Cas9, Cpfl, Cas12b, Cas12i, and Cas12j). A gene editing tool of the TALEN technology refers to a restriction enzyme capable of cleaving a specific DNA sequence, which includes a transcription activator-like (TAL) effector DNA binding domain and a DNA cleavage domain. A gene editing tool of the ZFN technology refers to a restriction enzyme that can cleave a specific DNA sequence, which includes a zinc-finger DNA binding domain and a DNA cleavage domain. It is well known to those skilled in the art that an intracellular genome can be edited by constructing nucleotides encoding a gene editing tool and other regulatory elements into a suitable vector and then transforming a reconstructed vector into a cell. A type of the editing includes gene knockout, insertion, and base editing.

As used herein, the term "gene editing enzyme" refers to a nuclease suitable for CRISPR, TALEN, ZFN, and other editing tools. Preferably, the gene editing enzyme is a CRISPR enzyme, also known as a Cas protein, including, but not limited to: a Cas9 protein, a Cas12 protein, a Cas13 protein, a Cas14 protein, a Csm1 protein, and an FDK1 protein. The Cas protein refers to a protein family that may exhibit varying structures depending on origins, such as SpCas9 derived from *Streptococcus pyogenes* and SaCas9 derived from *Staphylococcus aureus.* Cas proteins can be classified based on structural characteristics (such as domains). For example, a Cas12 family may include Cas12a (also known as Cpf1), Cas12b, Cas12c, Cas12i, etc. The Cas protein may be double-stranded or single-stranded or may have no cleavage activity. The Cas protein of the present disclosure may be a WT Cas protein or a mutant thereof. A mutation type of the mutant includes replacement, substitution, or deletion of an amino acid. The mutant may or may not alter an enzyme cleavage activity of the Cas protein. Those skilled in the art know that a variety of Cas proteins with nucleic acid cleavage activities have been reported in the art. The known proteins or engineered variants thereof all can achieve the function of the present disclosure, which is incorporated into the protection scope of the present disclosure by reference.

As used herein, the terms "gRNA", "mature crRNA", and "guide sequence" can be used interchangeably and have the meaning commonly understood by those skilled in the art.

### BX genes

A protein encoded by a BX13 gene is a 2-oxoglutarate-dependent dioxygenase. An amino acid sequence encoded by the BX13 gene in corn is shown in SEQ ID NO.: 1. A nucleotide sequence of the BX13 gene is shown in SEQ ID NO.: 3. A protein encoded by a BX14 gene is a methyltransferase. An amino acid sequence encoded by the BX14 gene in corn is shown in SEQ ID NO.: 2. A nucleotide sequence of the BX14 gene is shown in SEQ ID NO.: 4.

It should be understood that, although the genes provided in the embodiments of the present disclosure are derived from corn, a gene sequence that is derived from other similar plants and has a specified homology (for example, 70% or more, such as 70%, 75%, 80%, 85%, 90%, 95%, or even 98%, 99%, or 100%) with the sequence of the present disclosure (preferably a sequence shown in any of SEQ ID NOs.: 1-4) is also encompassed within the scope of the present disclosure, as long as those skilled in the art can readily isolate such a sequence from other plants based on the information provided by the present application after reading the present application. A method and a tool for aligning sequences to determine an identity are also well known in the art, such as BLAST.

The term "same or similar function" primarily refers to an activity of enhancing pest resistance of a plant, improving disease resistance of a plant, increasing an ear size, increasing a kernel size, increasing a hundred-kernel weight, or boosting a yield of a plant through a loss of an activity of a protein.

### Agricultural inhibitor

The active substance of the present disclosure (such as an inhibitor for the BX13 gene and/or the BX14 gene) can be prepared into an agricultural preparation by a conventional method, such as a solution, an emulsion, a suspension, a powder, a foam, a paste, a granule, an aerosol, a natural and synthetic material impregnated with the active substance, a microcapsule in a polymer, and a seed coating agent.

The formulation can be produced by a known method. For example, the active substance is mixed with an extender. The extender is a liquid, liquefied gas, or solid diluent or carrier, and can optionally be a surfactant, namely, an emulsifier and/or a dispersant and/or a foam-forming agent. For example, when water is adopted as the extender, an organic solvent can also be adopted as an additive.

The use of a liquid solvent as a diluent or a carrier is generally applicable, including aromatic hydrocarbons, such as xylene, toluene, or alkyl naphthalene; chlorinated aromatic hydrocarbons or chlorinated aliphatic hydrocarbons, such as chlorobenzene, vinyl chloride, or dichloromethane; aliphatic hydrocarbons, such as cyclohexane or paraffin, including mineral oil fractions; alcohols, such as ethanol or ethylene glycol and ethers and esters thereof; ketones, such as acetone, methyl ethyl ketone, methyl isobutyl ketone, or cyclohexanone; or less common polar solvents, such as dimethylformamide and dimethyl sulfoxide (DMSO), and water.

A liquefied gas diluent or carrier refers to a liquid that becomes a gas at room temperature and ambient pressure, for example, an aerosol propellant such as a halogenated hydrocarbon, and butane, propane, nitrogen, and carbon dioxide.

A solid carrier can be a ground natural mineral, such as kaolin, clay, talc, quartz, activated clay, montmorillonite, or diatomaceous earth, and a ground synthetic mineral, such as highly dispersed silica, alumina, and silicates. A solid carrier for granules is crushed and fractionated natural zircon, such as calcite, marble, pumice, sepiolite, and dolomite; particles synthesized from inorganic and organic coarse powders; and organic materials such as particles of sawdust, coconut shells, corn cobs, and tobacco stalks.

Nonionic and anionic emulsifiers may be adopted as emulsifiers and/or foam-forming agents, for example, polyoxyethylene-fatty acid esters and polyoxyethylene-fatty alcohol ethers, such as alkyl aryl polyglycol ethers, alkyl sulfonates, alkyl sulfates, aryl sulfonates, and albumin hydrolysates. The dispersant includes, for example, a lignin sulfite waste liquid and methyl cellulose.

A binder can be used in the formulation, such as carboxymethyl cellulose, and natural and synthetic polymers in a powder, granule, or emulsion form, including gum arabic, polyvinyl alcohol, and polyvinyl acetate.

A coloring agent can also be used, for example, an inorganic dye, such as iron oxide, cobalt oxide, and Prussian blue; an organic dye, such as an organic dye, such as an azo dye or a metal phthalocyanine dye; and a trace nutrient, such as salts of iron, manganese, boron, copper, cobalt, aluminum, and zinc.

In the present disclosure, the "agricultural preparation" typically refers to an agricultural plant growth regulator including: an inhibitor for the BX13 gene and/or the BX14 gene as an active ingredient for improving a trait of a plant (for example, enhancing pest resistance or disease resistance of the plant, increasing an ear size, increasing a kernel size, increasing a hundred-kernel weight, or boosting a yield of the plant), and an agriculturally acceptable carrier.

As used herein, the term "agriculturally acceptable carrier" refers to an agriculturally acceptable solvent, suspending agent, or excipient for delivering the active ingredient of the present disclosure to a plant. The carrier can be a liquid or a solid. The agriculturally acceptable carrier suitable for the present disclosure is selected from the group consisting of water, a buffer, DMSO, a surfactant such as Tween-20, or a combination thereof. Any agriculturally acceptable carrier known to those skilled in the art can be adopted in the present disclosure.

The agricultural inhibitor of the present disclosure may include an agricultural composition.

The agricultural preparation of the present disclosure may be used in combination with other substances for enhancing pest resistance or disease resistance of a plant, increasing an ear size, increasing a kernel size, increasing a hundred-kernel weight, or boosting a yield of the plant. The other substances for enhancing pest resistance or disease resistance of a plant, increasing an ear size, increasing a kernel size, increasing a hundred-kernel weight, or boosting a yield of the plant can be plant growth regulators known to those skilled in the art.

The agricultural preparation of the present disclosure can be in various dosage forms, as long as the active ingredient can be effectively delivered into a plant. From the perspective of easy preparation and application, the preferred agricultural preparation is a spray or solution formulation.

The agricultural preparation of the present disclosure generally includes the active ingredient of the present disclosure at a content of 0.0001 wt% to 99 wt% and preferably 0.1 wt% to 90 wt% of a total weight of the agricultural preparation. A concentration of the active ingredient of the present disclosure in a commercial formulation or a dosage form can vary within a wide range. The concentration of the active ingredient of the present disclosure in the commercial formulation or the dosage form can be 0.0000001% to 100% (g/v) and preferably 0.0001% to 50% (g/v).

### Major advantages of the present disclosure:

The present disclosure has found through research that the inhibition on an expression level or activity of a BX13 gene and a BX14 gene can enhance the pest resistance and disease resistance of a plant, increase the ear and kernel sizes of the plant, increase the hundred-kernel weight of the plant, or improve the yield of the plant.

### DESCRIPTION OF THE DRAWINGS

FIG. 1 is a schematic diagram of a gene-editing vector;
FIG. 2 shows growth curves of armyworms, where WT is for an armyworm fed with a WT corn plant, L1 is for an armyworm fed with a 74-3618 corn plant, L2 is for an armyworm fed with a 75-3672 corn plant, and L3 is for an armyworm fed with a 77-3740 corn plant;
FIG. 3 shows statuses of WT and gene-edited corn plants on which armyworms have been fed, where WT is for a WT corn plant and bx13/14 is for a corn plant in which both BX13 and BX14 are edited;
FIG. 4 shows internal infection conditions in corn stalks on day 12 after the corn stalks are injected with *Fusarium graminearum,* where corn stalk nodes shown in this figure, from top to bottom, include: a stalk node above an inoculation site, a stalk node at which the inoculation site is located, and a stalk node below the inoculation site;
FIG. 5 shows ear and kernel phenotypes of a WT female parent W67 of Wan Nuo 2000, and edited plants 74-3618, 75-3672, and 77-3740; and
FIG. 6 is a bar chart of hundred-kernel weight statistics of the WT female parent W67 of Wan Nuo 2000, and edited plants 74-3618 and 77-3740.

### SPECIFIC IMPLEMENTATIONS

The present disclosure will be further explained below in conjunction with examples. The following examples are only preferred examples of the present disclosure, and are not intended to limit the present disclosure in other forms. Any technical personnel familiar with the profession may use the technical content disclosed above to derive equivalent examples through equivalent changes. Any simple modification or equivalent change made to the following examples according to the technical essence of the present disclosure without departing from the content of the solutions of the present disclosure shall fall within the protection scope of the present disclosure.

### Example 1: Production of gene-edited corn

In this example, Cas9 and single-guide RNAs (sgRNAs) targeting BX13 and BX14 genes were used to edit the two genes in corn. A specific operational process can be a conventional process in the art. The BX13 gene corresponds to an accession number of Zm00001d007718, an amino acid sequence shown in SEQ ID No. 1, and a coding sequence (CDS) shown in SEQ ID No. 3. The BX14 gene corresponds to an accession number of Zm00001d004921, an amino acid sequence shown in SEQ ID No. 2, and CDS shown in SEQ ID No. 4.

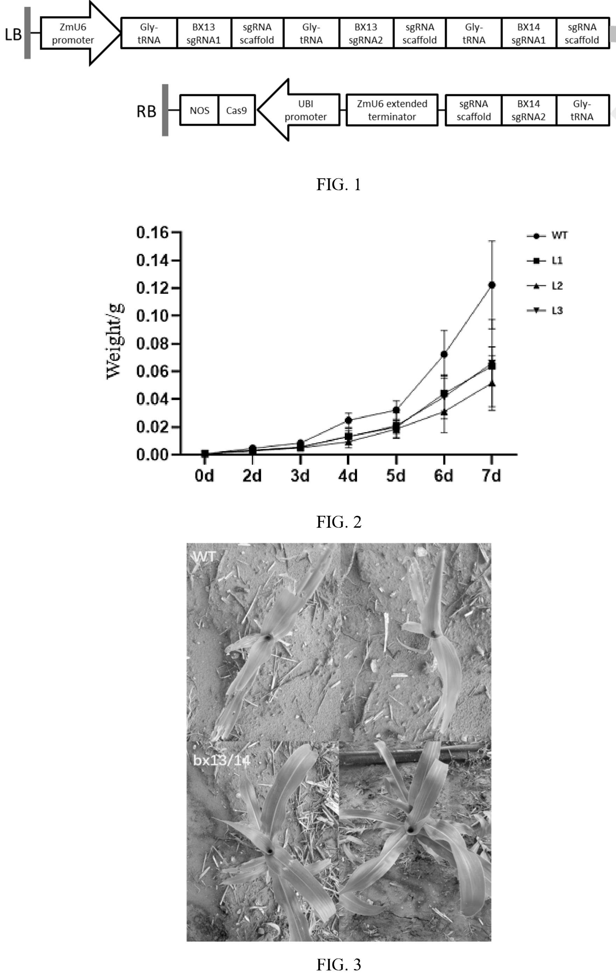

In this example, a schematic diagram of a constructed gene-editing vector is shown in FIG. 1, where a ZmU6 promoter represents a U6 promoter, Gly-tRNA represents glycine tRNA, and a UBI promoter represents an ubiquitin (UBI) promoter. Vector construction may refer to the reference ("High-efficiency CRISPR/Cas9 multiplex gene editing using the glycine tRNA-processing system-based strategy in maize", Weiwei Qi et al., *BMC Biotechnology,* 2016).
Amino acid sequence encoded by the BX13 gene: 
MAPTAAKDSGSADRWREVQAFEDSKLGVKGLVDSGVKSIPAMFHHPPESLKDVISPP
ALPSSDDAPAIPVVDLSVARREDLVAQVKHAAGTVGFFWVVNHGVPEELMASMLSGVR
QFNEGSLEAKQALYSRDPARNVRFASNFDLFESAAADWRDTLYCKIAPDPAPRELVPEPL
RNVMMEYGEELTKLARSMFELLSESLGMPSDHLHKMECMQQLHIVCQYYPPCPEPHLTI
GVRKHSDTGFFTILLQDGMGGLQVLVDRGGGRQTWVDVTPRPGALMVNMGSFLQLVT
NDRYKSVDHRVPANKSSDTARVSVAAFFNPDEKRTERLYGPIPDPSKPPLYRSVTFPDFIA
KFNSIGLDGRVLDHFRLEDDGPTRLAAPAHHV (SEQ ID No. 1).
Amino acid sequence encoded by the BX14 gene: 
MALMQESSQDLLEAHDELFHHCLCFAKSLALAVAQDLRIPDAIHHHGGATLHQILA
EAALHPSKLRALRRLMRVLTVSGVFTVQYSSTVDASDGADVVYRLTAASRFLVSDSDEA
GTASLAPFANLALHPIAISPHAVGICAWFRQEQHDPSPYGLAFRQIPTIWEHADNVNALLN
KGLLAESRFLMPIVLRECGDEVFRGIDSLVDVGGGHGGAAATIAAAFPHVKCSVLDLPH
VVAGAPSDACVQFVAGNMFHSIPPATAVFFKTTLCDWGDDECIKILKNCKQAISPRDEGG
KVIIMDVVVGYGQSNMKRLETQVMFDLVMMAVNGVERDEQEWKEMFIEAGFKDYKIR
PVAGLMSVIEVYP (SEQ ID No. 2).
Coding sequence (CDS) of the BX13 gene: 
ATGGCTCCGACCGCCGCCAAGGACAGTGGCTCCGCCGATCGCTGGCGCGAGGTG
CAGGCGTTCGAGGACAGCAAGCTGGGTGTGAAGGGCCTGGTCGACTCCGGCGTCAA
GTCCATCCCGGCCATGTTCCACCACCCGCCGGAGTCCCTCAAGGATGTCATTTCTCCA
CCAGCTCTGCCCTCCTCCGACGATGCGCCGGCCATCCCAGTGGTGGACCTGTCCGTG
GCGCGGCGCGAGGATCTGGTGGCCCAGGTGAAGCACGCGGCCGGGACGGTGGGCTT
 
CTTCTGGGTGGTGAACCACGGCGTGCCGGAGGAGCTGATGGCCAGCATGCTCAGCG
GGGTGCGCCAGTTCAACGAGGGCTCTCTGGAGGCCAAGCAGGCGCTCTACTCACGG
GACCCCGCTCGCAACGTGCGCTTCGCTTCCAACTTCGACCTCTTCGAGTCCGCGGCG
GCCGACTGGCGCGACACGCTCTACTGCAAGATTGCCGGATCCGGCGCCGCGGGA
GCTCGTGCCGGAGCCTCTCCGGAACGTGATGATGGAATACGGCGAGGAACTGACGA
AGCTGGCGCGGTCCATGTTCGAGCTGCTGTCGGAGTCCCTCGGCATGCCCAGCGACC
ACCTGCACAAGATGGAGTGCATGCAGCAGCTCCACATCGTGTGCCAGTACTACCCGC
CATGCCCGGAGCCGCACCTCACCATCGGCGTCAGGAAGCACTCGGACACCGGTTTCT
TCACCATCCTCCTGCAGGACGGCATGGGCGGCCTGCAGGTGCTAGTCGATCGCGGAG
GCGGCCGCCAGACGTGGGTGGACGTCACTCCCCGACCTGGGGCACTCATGGTCAACA
TGGGCAGCTTTCTTCAGCTTGTGACGAATGACCGGTACAAGAGCGTGGATCACCGGG
TGCCGGCTAACAAGAGCAGTGACACGGCGAGGGTCTCGGTAGCTGCCTTCTTCAACC
CCGACGAGAAGAGAACCGAGAGGCTGTATGGCCCCATTCCAGACCCCAGCAAGCCT
CCGCTGTACAGGAGCGTCACGTTCCCGGACTTCATCGCCAAGTTCAACAGCATCGGA
CTGGACGGTCGCGTGCTCGACCACTTCCGGTTGGAGGACGACGGCCCTACTCGTCTT
GCTGCTCCTGCACACCACGTCTAG (SEQ ID No. 3).
CDS of the BX14 gene:

ATGGCACTCATGCAGGAGAGCAGCCAGGACTTGCTCGAAGCGCACGACGAGCTC
TTCCACCACTGCCTGTGCTTCGCCAAATCGCTCGCGCTCGCCGTGGCGCAGGACCTC
CGCATCCCCGACGCGATCCACCACCACGGAGGCGGCGCCACCCTCCACCAGATCCTC
GCCGAGGCCGCGCTCCACCCAAGCAAGCTTCGCGCCCTACGCCGCCTGATGCGCGTG
CTCACCGTCTCGGGCGTCTTCACCGTCCAGTATTCTTCAACCGTCGACGCGTCGGACG
GAGCTGATGTCGTCTACAGGCTGACGGCAGCCTCCCGCTTCCTCGTCAGCGATAGCG
ACGAGGCGGGCACGGCGTCCTTGGCTCCCTTTGCGAACCTGGCGCTCCACCCTATCG
CCATCTCCCCGCACGCCGTGGGCATCTGCGCGTGGTTCCGGCAGGAGCAGCACGACC
CGTCCCCGTACGGCCTGGCGTTCCGCCAGATCCCGACCATCTGGGAGCATGCTGACA
ACGTAAACGCCCTACTGAACAAAGGCTTGCTCGCGGAAAGCCGCTTCTTGATGCCAA
TCGTACTCAGGGAGTGCGGAGACGAGGTGTTCCGTGGGATCGACTCGTTGGTCGACG
TCGGCGGTGGGCACGGTGGCGCCGCCGCCACCATCGCCGCCGCATTCCCGCACGTCA
AGTGCAGCGTGCTTGACCTCCCGCACGTTGTCGCCGGTGCTCCATCCGATGCCTGCGT
GCAGTTCGTTGCGGGCAATATGTTCCACAGTATTCCACCTGCAACCGCCGTTTTCTTC
AAGACAACTCTATGTGACTGGGGTGACGACGAGTGCATCAAGATATTGAAGAATTGC
AAGCAAGCCATATCTCCACGGGATGAGGGTGGAAGGTGATAATCATGGACGTGGTA
GTCGGGTATGGGCAGTCAAACATGAAGCGCCTAGAGACACAAGTTATGTTTGATTTG
GTTATGATGGCGGTCAATGGAGTCGAGCGCGACGAGCAAGAGTGGAAGGAGATGTTC

ATTGAAGCTGGATTCAAAGACTACAAAATCCGACCAGTAGCTGGCCTCATGTCGGTCA
TCGAGGTCTATCCATGA (SEQ ID No. 4).

In this example, sgRNAs targeting the BX13 gene and the BX14 gene were designed using the Target Design (http://skl.scau.edu.cn/targetdesign/). The sgRNA sequences are listed in Table 1.

**Table 1 Information on the sgRNA sequences**

| sgRNA name | sgRNA sequence (5'-3') |
|---|---|
| *BX13 s*gRNA1 | ATGACATCCTTGAGGGACTC |
| *BX13 s*gRNA2 | CCAAGCAGGCGCTCTACTCA |
| *BX14 s*gRNA1 | CTTGGCTCCCTTTGCGAACC |
| *BX14 s*gRNA2 | CTTGATGCCAATCGTACTCA |

The constructed vector was transformed into *Agrobacterium,* and transformed *Agrobacterium* was used to infect corn immature embryos. Infected corn immature embryos were subjected to cultivation, screening, differentiation, rooting, and growth to produce complete plants. Editing patterns of gene-edited corn plants were then confirmed using primers listed in Table 2 (a corn variety used in this experiment was a WT female parent W67 of Wan Nuo 2000).

**Table 2 Information on primer sequences used for detecting editing types**

| Primer name | Primer sequence (5'-3') |
|---|---|
| BX13-SNP-F | agaccgtatcgtggttgcag |
| BX13-SNP-R | cgatctacacaccggagagg |
| BX14-SNP-F | tcgaacacgacacgcgataa |
| BX14-SNP-R | agttgtctggcagtcgatcag |

With the above method, corn plants 74-3618, 75-3672, and 77-3740 with both BX13 and BX14 genes edited were produced. Editing types of these corn plants are shown in Tables 3 to 6, which are specifically as follows:
Editing results for the edited plant 74-3618: 242 nucleotides after nucleotide 144 in CDS of the BX13 gene (SEQ ID No. 3) are deleted, which leads to a frameshift mutation in the subsequent coding region and finally results in the premature termination of translation of a corresponding protein. 2 nucleotides A and C after nucleotide 572 in CDS of the BX14 gene (SEQ ID No. 4) are deleted, which leads to a frameshift mutation subsequently and finally results in the premature termination of translation of a corresponding protein. In the edited plant 74-3618, CDS of a BX13 gene is shown in SEQ ID No. 5, and CDS of a BX14 gene is shown in SEQ ID No. 6.
Editing results for the edited plant 75-3672: 242 nucleotides after nucleotide 144 in CDS of the BX13 gene (SEQ ID No. 3) are deleted, which leads to a frameshift mutation in the subsequent coding region and finally results in the premature termination of translation of a corresponding protein. 197 nucleotides after nucleotide 376 in CDS of the BX14 gene (SEQ ID No. 4) are deleted, which leads to a frameshift mutation subsequently and finally results in the premature termination of translation of a corresponding protein. In the edited plant 75-3672, CDS of a BX13 gene is shown in SEQ ID No. 5, and CDS of a BX14 gene is shown in SEQ ID No. 8.
Editing results for the edited plant 77-3740: One nucleotide T after nucleotide 145 in CDS of the BX13 gene (SEQ ID No. 3) is added, which leads to a frameshift mutation in the subsequent coding region and finally results in the premature termination of translation of a corresponding protein. 2 nucleotides A and C after nucleotide 572 in CDS of the BX14 gene (SEQ ID No. 4) are deleted, which leads to a frameshift mutation subsequently and finally results in the premature termination of translation of a corresponding protein. In the edited plant 77-3740, CDS of a BX13 gene is shown in SEQ ID No. 7, and CDS of a BX14 gene is shown in SEQ ID No. 6.

**Table 3 Sequence alignment of BX13 genes at sgRNA1 in different edited plants**

| Plant name | BX13 sgRNA1 target region (5'-3') | Mutation type |
|---|---|---|
| W67 | CCGCCGGAGTCCCTCAAGGATGTCATTTCT | WT |
| 74-3618 | CCGCCGGAG- - - - - - - - - - - - - - - - - - - - - - - | -242bp |
| 75-3672 | CCGCCGGAG- - - - - - - - - - - - - - - - - - - - - - - | -242bp |
| 77-3740 | CCGCCGGAGTTCCCTCAAGGATGTCATTTCT | +T |

**Table 4 Sequence alignment of BX13 genes at sgRNA2 in different edited plants**

| Plant name | BX13 sgRNA2 target region (5'-3') | Mutation type |
|---|---|---|
| W67 | AGGCCAAGCAGGCGCTCTACTCACGGGACC | WT |
| 74-3618 | - - - - - - - - - - - - - - - - - - - - - - CTCACGGGACC | -242bp |
| 75-3672 | - - - - - - - - - - - - - - - - - - - - - - CTCACGGGACC | -242bp |
| 77-3740 | AGGCCAAGCAGGCGCTCTACTCACGGGACC | WT |

**Table 5 Sequence alignment of BX14 genes at sgRNA1 in different edited plants**

| Plant name | BX14 sgRNA1 target region (5'-3') | Mutation type |
|---|---|---|
| W67 | GGCGTCCTTGGCTCCCTTTGCGAACCTGGC | WT |
| 74-3618 | GGCGTCCTTGGCTCCCTTTGCGAACCTGGC | WT |
| 75-3672 | GGCGTCCTTGGCTCCCTTTGCGA - - - - - - - - | -197bp |
| 77-3740 | GGCGTCCTTGGCTCCCTTTGCGAACCTGGC | WT |
| Table 6 Sequence alignment of BX14 genes at sgRNA2 in different edited plants | | |

| Plant name | BX14 sgRNA2 target region (5'-3') | Mutation type |
|---|---|---|
| W67 | CCGCTTCTTGATGCCAATCGTACTCAGGGA | WT |
| 74-3618 | CCGCTTCTTGATGCCAATCGT- -TCAGGGA | -AC |
| 75-3672 | - - - - - - - - - - - - - - - - - - - - - - - - CTCAGGGA | -197bp |
| 77-3740 | CCGCTTCTTGATGCCAATCGT- -TCAGGGA | -AC |

| | | |
|---|---|---|
| Note: An underlined portion represents a protospacer adjacent motif (PAM) sequence. | | |

CDS of BX13 genes in the edited plants 74-3618 and 75-3672: 
ATGGCTCCGACCGCCGCCAAGGACAGTGGCTCCGCCGATCGCTGGCGCGAGGTG
CAGGCGTTCGAGGACAGCAAGCTGGGTGTGAAGGGCCTGGTCGACTCCGGCGTCAA
GTCCATCCCGGCCATGTTCCACCACCCGCCGGAGCTCACGGACCCCGCTCGCAACG
TGCGCTTCGCTTCCAACTTCGACCTCTTCGAGTCCGCGGCGGCCGACTGGCGCGACA
CGCTCTACTGCAAGATTGCGCCGGATCCGGCGCCGCGGAGCTCGTGCCGGAGCCTC
TCCGGAACGTGATGATGGAATACGGCGAGGAACTGACGAAGCTGGCGCGGTCCATGT
TCGAGCTGCTGTCGGAGTCCCTCGGCATGCCCAGCGACCACCTGCACAAGATGGAGT
GCATGCAGCAGCTCCACATCGTGTGCCAGTACTACCCGCCATGCCCGGAGCCGCACC
TCACCATCGGCGTCAGGAAGCACTCGGACACCGGTTTCTTCACCATCCTCCTGCAGG
ACGGCATGGGCGGCCTGCAGGTGCTAGTCGATCGCGGAGGCGGCCGCCAGACGTGG
GTGGACGTCACTCCCCGACCTGGGGCACTCATGGTCAACATGGGCAGCTTTCTTCAG
CTTGTGACGAATGACCGGTACAAGAGCGTGGATCACCGGGTGCCGGCTAACAAGAG
CAGTGACACGGCGAGGGTCTCGGTAGCTGCCTTCTTCAACCCCGACGAGAAGAGAA
CCGAGAGGCTGTATGGCCCCATTCCAGACCCCAGCAAGCCTCCGCTGTACAGGAGCG
TCACGTTCCCGGACTTCATCGCCAAGTTCAACAGCATCGGACTGGACGGTCGCGTGC
TCGACCACTTCCGGTTGGAGGACGACGGCCCTACTCGTCTTGCTGCTCCTGCACACC
ACGTCTAG (SEQ ID No. 5).
CDS of BX14 genes in the edited plants 74-3618 and 77-3740: 
ATGGCACTCATGCAGGAGAGCAGCCAGGACTTGCTCGAAGCGCACGACGAGCTC
TTCCACCACTGCCTGTGCTTCGCCAAATCGCTCGCGCTCGCCGTGGCGCAGGACCTC
CGCATCCCCGACGCGATCCACCACCACGGAGGCGGCGCCACCCTCCACCAGATCCTC
GCCGAGGCCGCGCTCCACCCAAGCAAGCTTCGCGCCCTACGCCGCCTGATGCGCGTG
CTCACCGTCTCGGGCGTCTTCACCGTCCAGTATTCTTCAACCGTCGACGCGTCGGACG
GAGCTGATGTCGTCTACAGGCTGACGGCAGCCTCCCGCTTCCTCGTCAGCGATAGCG
ACGAGGCGGGCACGGCGTCCTTGGCTCCCTTTGCGAACCTGGCGCTCCACCCTATCG
 
CCATCTCCCCGCACGCCGTGGGCATCTGCGCGTGGTTCCGGCAGGAGCAGCACGACC
CGTCCCCGTACGGCCTGGCGTTCCGCCAGATCCCGACCATCTGGGAGCATGCTGACA
ACGTAAACGCCCTACTGAACAAAGGCTTGCTCGCGGAAAGCCGCTTCTTGATGCCAA
TCGTTCAGGGAGTGCGGAGACGAGGTGTTCCGTGGGATCGACTCGTTGGTCGACGTC
GGCGGTGGGCACGGTGGCGCCGCCGCCACCATCGCCGCCGCATTCCCGCACGTCAAG
TGCAGCGTGCTTGACCTCCCGCACGTTGTCGCCGGTGCTCCATCCGATGCCTGCGTGC
AGTTCGTTGCGGGCAATATGTTCCACAGTATTCCACCTGCAACCGCCGTTTTCTTCAA
GACAACTCTATGTGACTGGGGTGACGACGAGTGCATCAAGATATTGAAGAATTGCAA
GCAAGCCATATCTCCACGGGATGAGGGTGGAAGGTGATAATCATGGACGTGGTAGT
CGGGTATGGGCAGTCAAACATGAAGCGCCTAGAGACACAAGTTATGTTTGATTTGGTT
ATGATGGCGGTCAATGGAGTCGAGCGCGACGAGCAAGAGTGGAAGGAGATGTTCATT
GAAGCTGGATTCAAAGACTACAAAATCCGACCAGTAGCTGGCCTCATGTCGGTCATC
GAGGTCTATCCATGA (SEQ ID No. 6).
CDS of a BX13 gene in the edited plant 77-3740: 
ATGGCTCCGACCGCCGCCAAGGACAGTGGCTCCGCCGATCGCTGGCGCGAGGTG
CAGGCGTTCGAGGACAGCAAGCTGGGTGTGAAGGGCCTGGTCGACTCCGGCGTCAA
GTCCATCCCGGCCATGTTCCACCACCCGCCGGAGTTCCCTCAAGGATGTCATTTCTCC
ACCAGCTCTGCCCTCCTCCGACGATGCGCCGGCCATCCCAGTGGTGGACCTGTCCGT
GGCGCGGCGCGAGGATCTGGTGGCCCAGGTGAAGCACGCGGCCGGGACGGTGGGCT
TCTTCTGGGTGGTGAACCACGGCGTGCCGGAGGAGCTGATGGCCAGCATGCTCAGCG
GGGTGCGCCAGTTCAACGAGGGCTCTCTGGAGGCCAAGCAGGCGCTCTACTCACGG
GACCCCGCTCGCAACGTGCGCTTCGCTTCCAACTTCGACCTCTTCGAGTCCGCGGCG
GCCGACTGGCGCGACACGCTCTACTGCAAGATTGCGCCGGATCCGGCGCCGCGGGA
GCTCGTGCCGGAGCCTCTCCGGAACGTGATGATGGAATACGGCGAGGAACTGACGA
AGCTGGCGCGGTCCATGTTCGAGCTGCTGTCGGAGTCCCTCGGCATGCCCAGCGACC
ACCTGCACAAGATGGAGTGCATGCAGCAGCTCCACATCGTGTGCCAGTACTACCCGC
CATGCCCGGAGCCGCACCTCACCATCGGCGTCAGGAAGCACTCGGACACCGGTTTCT
TCACCATCCTCCTGCAGGACGGCATGGGCGGCCTGCAGGTGCTAGTCGATCGCGGAG
GCGGCCGCCAGACGTGGGTGGACGTCACTCCCCGACCTGGGGCACTCATGGTCAACA
TGGGCAGCTTTCTTCAGCTTGTGACGAATGACCGGTACAAGAGCGTGGATCACCGGG
TGCCGGCTAACAAGAGCAGTGACACGGCGAGGGTCTCGGTAGCTGCCTTCTTCAACC
CCGACGAGAAGAGAACCGAGAGGCTGTATGGCCCCATTCCAGACCCCAGCAAGCCT
CCGCTGTACAGGAGCGTCACGTTCCCGGACTTCATCGCCAAGTTCAACAGCATCGGA
CTGGACGGTCGCGTGCTCGACCACTTCCGGTTGGAGGACGACGGCCCTACTCGTCTT
GCTGCTCCTGCACACCACGTCTAG (SEQ ID No. 7).
CDS of a BX14 gene in the edited plant 75-3672:

ATGGCACTCATGCAGGAGAGCAGCCAGGACTTGCTCGAAGCGCACGACGAGCTC
TTCCACCACTGCCTGTGCTTCGCCAAATCGCTCGCGCTCGCCGTGGCGCAGGACCTC
CGCATCCCCGACGCGATCCACCACCACGGAGGCGGCGCCACCCTCCACCAGATCCTC
GCCGAGGCCGCGCTCCACCCAAGCAAGCTTCGCGCCCTACGCCGCCTGATGCGCGTG
CTCACCGTCTCGGGCGTCTTCACCGTCCAGTATTCTTCAACCGTCGACGCGTCGGACG
GAGCTGATGTCGTCTACAGGCTGACGGCAGCCTCCCGCTTCCTCGTCAGCGATAGCG
ACGAGGCGGGCACGGCGTCCTTGGCTCCCTTTGCGACTCAGGGAGTGCGGAGACGA
GGTGTTCCGTGGGATCGACTCGTTGGTCGACGTCGGCGGTGGGCACGGTGGCGCCGC
CGCCACCATCGCCGCCGCATTCCCGCACGTCAAGTGCAGCGTGCTTGACCTCCCGCA
CGTTGTCGCCGGTGCTCCATCCGATGCCTGCGTGCAGTTCGTTGCGGGCAATATGTTC
CACAGTATTCCACCTGCAACCGCCGTTTTCTTCAAGACAACTCTATGTGACTGGGGTG
ACGACGAGTGCATCAAGATATTGAAGAATTGCAAGCAAGCCATATCTCCACGGGATG
AGGGTGGGAAGGTGATAATCATGGACGTGGTAGTCGGGTATGGGCAGTCAAACATGA
AGCGCCTAGAGACACAAGTTATGTTTGATTTGGTTATGATGGCGGTCAATGGAGTCGA
GCGCGACGAGCAAGAGTGGAAGGAGATGTTCATTGAAGCTGGATTCAAAGACTACA
AAATCCGACCAGTAGCTGGCCTCATGTCGGTCATCGAGGTCTATCCATGA (SEQ ID No.
8).

### Example 2: Pest resistance of gene-edited corn

Feeding experiment: A WT corn plant (WT female parent W67 of Wan Nuo 2000) and corn plants with both BX13 and BX14 genes edited (namely, the edited plants 74-3618, 75-3672, and 77-3740) were cultivated. A pest resistance assay was conducted using armyworm larvae (*Mythimna separata* walker, a chewing pest). Larvae in prominent and consistent growth conditions were selected, and initial weights of the selected larvae were measured by a balance and recorded. The weighed larvae were then individually placed in different ventilated and light-transmitting rearing boxes, and fed with leaves from the different plants. Young leaves were collected from the different plants at a same site for the feeding. Subsequently, a weight of each larva was measured and recorded daily. Throughout the experiment, it was ensured that there was always an adequate supply of edible leaves in each rearing box to support the normal growth of larvae.

Results of the feeding experiment are shown in FIG. 2. Compared with the WT corn plant (WT in FIG. 2), armyworms fed with leaves from the edited plants 74-3618 (L1 in FIG. 2), 75-3672 (L2 in FIG. 2), and 77-3740 (L3 in FIG. 2) demonstrate a slow weight gain and a low growth rate. That is, the corn plants with both BX13 and BX14 genes edited (namely, the edited plants 74-3618, 75-3672, and 77-3740) exhibit enhanced resistance to armyworms, indicating improved pest resistance.

Armyworms are inoculated onto corn plants growing in the field for feeding, and then a status of each corn plant was observed. Results are shown in FIG. 3. Compared to the WT corn plant W67 (WT in FIG. 3), the edited corn plant 74-3618 with both BX13 and BX14 genes edited (bx13/14 in FIG. 3) shows high leaf integrity and undergoes significantly-reduced damage from pest infestation, which further proves that the edited corn plant with both BX13 and BX14 genes edited has enhanced pest resistance.

Therefore, the mutation-induced inactivation of the BX13 gene and the BX14 gene can enhance the pest resistance of a plant, and can particularly improve the resistance of a plant to armyworms.

### Example 3: Stalk rot resistance of gene-edited corn

*Fusarium graminearum* was used to evaluate the resistance of a WT corn plant (WT female parent W67 of Wan Nuo 2000) and corn plants with both BX13 and BX14 genes edited (namely, the edited plants 74-3618 and 77-3740). Main steps were as follows:
(1) Culture of a pathogen spore suspension:
   *Fusarium graminearum* growing on a potato dextrose agar (PDA) medium was inoculated into a liquid medium, and cultured for approximately 72 h at 28°C under shaking at 200 rpm in the dark. After the culturing was completed, a resulting spore suspension was filtered through a double-layer gauze, and then concentrated or diluted to a working concentration of about 10⁷ spores/mL.
(2) Inoculation assay:
   The inoculation was conducted before flowering of corn plants. A 3 cm-long and 0.5 cm-deep wound was created with a scalpel in a middle of a 4th stalk node of a plant near the ground. 2 mL of the spore suspension was drawn with a syringe and injected at an angle of 45° downward into the wound (a WT corn plant injected with a same volume of water instead of the spore suspension was set as a negative control group). After the inoculation, watering was conducted once in the field. Then, routine fertilizer and water management was applied. 12 d after pathogen inoculation, internal parts of stalks of both the WT corn plant and the corn plants with both BX13 and BX14 genes edited were photographed to assess a disease infection degree.

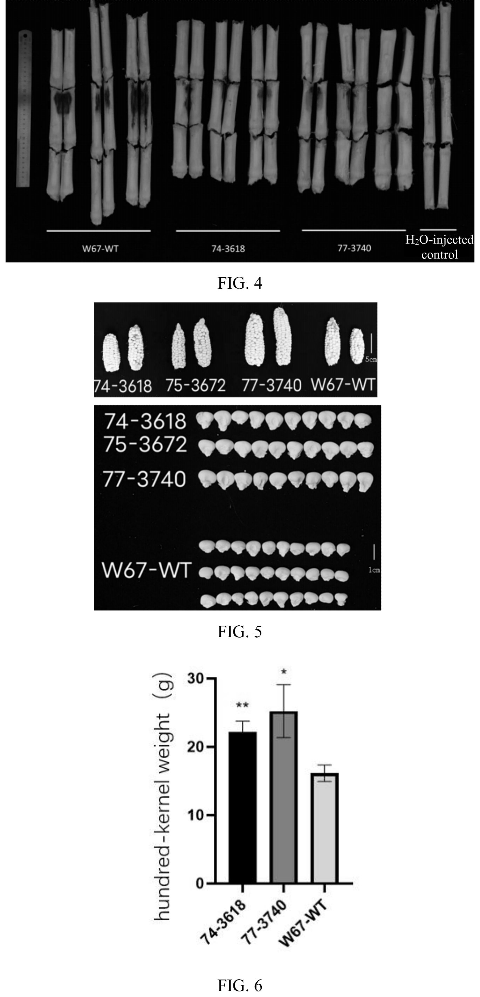

Results are shown in FIG. 4. A stalk in the negative control group (H₂O-injected control in FIG. 4) has no obvious bacterial plaque or disease symptom. A stalk of the WT corn plant inoculated with the pathogen (W67-WT in FIG. 4) is significantly infected, with a maximum bacterial plaque area. Compared to a stalk of the WT corn plant, stalks of the corn plants with both BX13 and BX14 genes edited (74-3618 and 77-3740 in FIG. 4) demonstrate a low degree of infection, which is characterized by a light color and a small area of a bacterial plaque. It can be seen that the editing of both BX13 and BX14 genes can enhance the resistance of corn to stalk rot. The mutation-induced inactivation of the BX13 gene and the BX14 gene can improve the disease resistance of a plant, and can particularly improve the resistance of a plant to stalk rot.

### Example 4: Investigation of agronomic traits of gene-edited corn

A WT corn plant (WT female parent W67 of Wan Nuo 2000) and BX13/BX14-edited corn plants (namely, the edited plants 74-3618, 75-3672, and 77-3740) were planted in the field, and routine water and fertilizer management was applied. 45 d after manual pollination, Corn cobs were harvested, oven-dried, and threshed. Phenotypes of an ear size, a kernel size, and a hundred-kernel weight were then analyzed.

Analysis results for the ear and kernel sizes are shown in FIG. 5. The WT corn plant (W67-WT in FIG. 5) has relatively small ear and kernel sizes (due to the inbred line W67). Compared with the WT corn plant, the BX13/BX14-edited corn plants 74-3618, 75-3672, and 77-3740 have significantly-increased ear and kernel sizes.

Analysis results for the hundred-kernel weight are shown in FIG. 6. Compared with the WT corn plant (W67-WT in FIG. 6), the BX13/BX14-edited corn plants 74-3618 and 77-3740 have a significantly-increased hundred-kernel weight.

It can be seen that the mutation-induced inactivation of the BX13 gene and the BX14 gene can increase an ear size, a kernel size, and a hundred-kernel weight, thereby improving a yield.

All documents mentioned in the present disclosure are cited as references in the present application, as if each document was individually cited as a reference. In addition, it should be understood that various changes or modifications may be made to the present disclosure by those skilled in the art after reading the above teaching content of the present disclosure, and these equivalent forms also fall within the scope defined by the appended claims of the present disclosure.

## Claims

1. A method for improving a trait of a plant, comprising a step of mutating a BX13 gene and/or a BX14 gene,
wherein an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

2. The method according to claim 1, wherein improving the trait of the plant is selected from one or more of the following (i) to (vi):
(i) enhancing pest resistance of the plant;
(ii) enhancing disease resistance of the plant;
(iii) increasing an ear size;
(iv) increasing a kernel size;
(v) increasing a hundred-kernel weight; and
(vi) boosting a yield of the plant.

3. The method according to claim 2, wherein enhancing the pest resistance of the plant comprises an increase in resistance of the plant to a chewing pest; and enhancing the disease resistance of the plant comprises an increase in resistance of the plant to stalk rot.

4. The method according to any one of claims 1 to 3, wherein the plant is a monocotyledonous plant; and
preferably, the plant is corn.

5. A method for producing a plant cell or a plant seed or a plant tissue or a plant part or a plant with an improved trait, comprising the following step:
reducing or inhibiting an expression level and/or activity of a BX13 gene and/or a BX14 gene in a plant cell or a plant seed or a plant tissue or a plant part or a plant,
wherein an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

6. The method according to claim 5, wherein reducing or inhibiting the expression level and/or the activity of the BX13 gene and/or the BX14 gene is achieved by a technology selected from the group consisting of gene mutation, gene knockout, gene disruption, an RNA interference technology, a gene editing technology, introduction of an inhibitor for a gene or a protein, or a combination thereof.

7. A method for producing a plant with an improved trait, comprising the following step:
regenerating the plant from the plant cell or the plant seed or the plant tissue or the plant part produced by the method according to any one of claims 5 to 6, so as to obtain the plant with the improved trait,
wherein preferably, the method further comprises a step of harvesting a plant seed from the plant with the improved trait.

8. A method for producing a hybrid plant, comprising a step of crossing the plant with the improved trait produced by the method according to claim 7 with another plant to produce the hybrid plant.

9. A use of a composition in improvement of a trait of a plant or in preparation of a reagent or a kit for producing a plant with an improved trait,
wherein the composition comprises:
(a) an inhibitor for a BX13 gene and/or a BX14 gene; and optionally,
(b) an agriculturally acceptable carrier;
an amino acid sequence encoded by the BX13 gene has a sequence identity of at least 70% with SEQ ID NO.: 1; and an amino acid sequence encoded by the BX14 gene has a sequence identity of at least 70% with SEQ ID NO.: 2.

10. The use according to claim 9, wherein the inhibitor is selected from the group consisting of a gene editing reagent, an antisense nucleic acid, an antibody, a small-molecule compound, a Crispr reagent, a small-molecule ligand, or a combination thereof.
